(19) Europäisches Patentamt
European Patent Office
Office européen des brevets

(11) Veröffentlichungsnummer : **0 006 252**
**B1**

(12) # EUROPÄISCHE PATENTSCHRIFT

(45) Veröffentlichungstag der Patentschrift : **21.03.84**

(51) Int. Cl.³ : **C 09 D 7/00**, C 09 D 5/04, C 08 G 18/00

(21) Anmeldenummer : **79200226.3**

(22) Anmeldetag : **09.05.79**

(54) **Verfahren zur Herstellung eines Thixotropiemittels.**

(30) Priorität : **26.05.78 DE 2822908**

(43) Veröffentlichungstag der Anmeldung : **09.01.80 Patentblatt 80/01**

(45) Bekanntmachung des Hinweises auf die Patenterteilung : **21.03.84 Patentblatt 84/12**

(84) Benannte Vertragsstaaten : **BE CH FR GB IT LU NL SE**

(56) Entgegenhaltungen :
**DE-A- 2 359 923**
**DE-A- 2 359 929**

(73) Patentinhaber : **Byk-Mallinckrodt Chemische Produkte GmbH**
**Mallinckrodtstrasse 5**
**D-4230 Wesel (DE)**

(72) Erfinder : **Haubennestel, Karl-Heinz**
**Herzog Adolf Strasse 36**
**D-4230 Wesel 1 (DE)**
Erfinder : **Mehren, Rainer, Dr. Dipl.-Chem.**
**Sonnenstrasse 3a**
**D-4230 Wesel 1 (DE)**

(74) Vertreter : **Frühbuss, Heinrich, Dr.rer.nat.**
**Franz-Fischer-Weg 61**
**D-4300 Essen 13 (DE)**

**EP 0 006 252 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Verfahren zur Herstellung eines Thixotropiemittels

Die bekannten Mittel zur Thixotropierung z. B. von Überzugsmitteln, wie Anstrichstoffe und Beschichtungsmassen bestehen aus hydriertem Rizinusöl, organischmodifizierten Bentoniten, hydrophilen Kieselsäuren und Metallseifen. Nachteilig bei diesen Mitteln ist, daß durch die meist festen Produkte Filmstörungen durch Seeding bzw. Mattierung auftreten können ; in bestimmten Lacksystemen wie Vinylharzlacken können auch Haftungsschwierigkeiten auftreten.

Als thixotropierende Mittel sind auch Polyamid-Harze mit freien Amino-Gruppen bekannt. Diese Produkte werden in die Lackharze mit eingebaut und können nicht nachträglich zur Korrektur den Lacken zugesetzt werden. Außerdem werden die auf diese Weise erzeugten Gele durch niedermolekulare Alkohole zerstört.

Ebenfalls ist bekannt, daß aus der Umsetzung von primären oder sekundären Aminen mit Mono- oder Di-Isocyanaten resultierende Harnstoffe, die *in situ* mit dem Bindemittel oder einem Teil des Bindemittels erzeugt werden, thixotropiegebend sind (DE-AS 2 359 923 und DE-AS 2 360 019).

Modifizierte Harnstoffe werden nach der DE-PS 1 805 693 auch zur Erzeugung von thixotropen Lösungsmittelgelen verwendet, die dann in Bindemittelformulierungen zur Thixotropieeinstellung verwendet werden.

Weiterhin ist aus der DE-AS 1 117 801 bekannt, daß Reaktionsprodukte von Mono- oder Polycarbonsäuren mit Isocyanaten bei der Kochung von Alkydharzen als thixotropiegebende Komponenten zugesetzt werden. Die in diesen Schriften angegebenen Verfahren haben den Nachteil, daß auch hier die Thixotropierung in einem Teil des Bindemittels oder Lösungsmittels erfolgen muß, da eine Reaktion der Ausgangskomponenten in hoher Konzentration (> 20 %) oder homogener Phase zu unlöslichen oder nicht mehr handhabbaren Produkten führt. Außerdem werden hier gewerbe-hygienisch nicht unbedenkliche Amine und Isocyanate den Überzugsmitteln zugesetzt. Diese Amine und Isocyanate können auch aufgrund ihrer eigenen hohen Reaktivität unerwünschte Reaktionen mit dem Bindemittel oder den Pigmenten eingehen.

Die vorliegende Erfindung hat sich die Aufgabe gestellt, ein thixotropiegebendes Mittel verfügbar zu machen, das in hoher Konzentration thixotropiegebende Gruppen aufweist, sich selbst aber in einem Zustand latenter Thixotropie befindet, flüssig und gewerbehygienisch unbedenklich ist, den Überzugsmitteln bei der Verarbeitung oder nachträglich zugesetzt werden kann und dort erst die gewünschte Thixotropie entfaltet.

Dieses Mittel wird dadurch hergestellt, dass vorzugsweise verzweigte Monohydroxyverbindungen ROH mit Diisocyanaten OCN—R'—NCO in Abwesenheit eines Bindemittels umgesetzt und die gebildeten Monoisocyanat-Addukte mit Polyaminen der Formel $H_2N—R''—NH_2$ in aprotischen Lösungsmitteln in Gegenwart von LiCl zu Harnstoffurethanen der Formel :

R—O—CO—NH—R'—NH—CO—NH—R''—NH—CO—NH—R'—NH—CO—OR

R = $C_nH_{2n+1}$- und $C_mH_{2m+1}(C_pH_{2p}O)_r$-
n = 4-22 ; m = 1-18 ; p = 2-4 ; r = 1-10

R' = 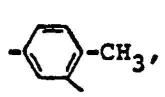, 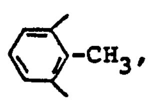, 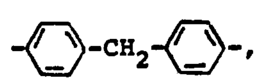, $-(CH_2)_6-$

R'' = 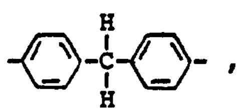, 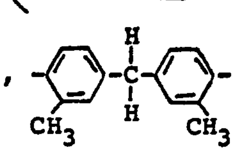,

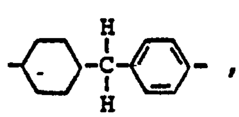, 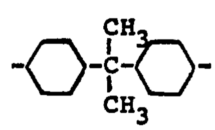

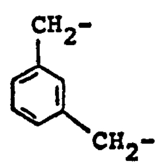

$-CH_2-CH_2-$ , $-(CH_2)_6-$ , $-(CH_2)_{12}-$

umgesetzt werden, wobei der Feststoffgehalt der gebildeten Lösung 10-75 Gew.% beträgt.

Diese erfindungsgemässen Mittel sollen einen Festkörpergehalt von vorzugsweise 40-60 Gew.%

aufweisen. Gibt man erfindungsgemässe Mittel in eine Überzugsformulierung, so treten an die Stelle des polaren aprotischen Lösungsmittels übliche Lacklösungsmittel, wie Alkohole, KW-Gemische, Ketone und Ester und es bildet sich ein Gelgerüst, das selbst in niedriger Anwendungskonzentration erhalten bleibt. Die Sol-Gelumwandlung kann beliebig oft wiedernolt werden, gleichgültig ob der Abbau des Gelzustandes thermisch oder mechanisch erfolgte.

Da das erfindungsgemässe Thixotropierungsmittel unterschiedliche Substituenten in seiner Harnstoffurethankomponente aufweisen kann, ist es möglich, seine Verträglichkeit an die jeweilige Polarität des zu thixotropierenden Überzugsmittelsystems anzupassen.

Für die Herstellung der erfindungsgemässen Mittel geht man zweckmäßigerweise von Mono-hydroxy-Verbindungen aus, die mit Di-Isocyanaten nach bekannten Verfahren zum Mono-Isocyanataddukt umgesetzt werden. Diese Mono-isocyanat-Addukte werden erfindungsgemäß in aprotischen Lösungsmitteln in Gegenwart von LiCl mit Polyaminen zu Harnstoffurethanen umgesetzt.

Als Mono-hydroxy-Verbindungen kommen geradkettige oder verzweigte aliphatische oder cyclische primäre oder sekundäre Alkohole mit 5-24 C-Atomen sowie Alkoxylierungsprodukte von Mono-hydroxy-Verbindungen in Frage. Vorzugsweise werden verzweigte Alkohole eingesetzt, um einer Kristallisation der substituierten Harnstoffurethane in Lösung vorzubeugen.

Für die Umsetzung der Mono-hydroxy-Verbindungen mit Di-Isocyanaten kommen handelsübliche Di-Isocyanate aliphatischer und aromatischer Natur in Frage, wie 1,6-Hexamethylen-di-isocyanat und 4,4-Diisocyanato-diphenylmethan. Besonders geeignet sind im Sinne der Erfindung die bekannten Toluylendiisocyanate in ihren isomeren Formen (2,4 und 2,6 Isomere).

Für die weitere Umsetzung im Sinne der Erfindung eignen sich insbesondere primäre und/oder sekundäre aliphatische, cycloaliphatische oder aromatische Amine, die mindestens 2 mit Isocyanat reagierende Aminogruppen aufweisen, wie 4,4-Diamino-diphenylmethan, 3,3-Dimethyl-4,4-diaminodiphenylmethan, 2,2-Bis(4-aminocyclohexyl)-propan, N,N-Dimethyl-4,4-diaminodiphenylmethan, (3-Methyl-4-aminocyclohexyl)-(3-Methyl-4-aminophenyl)-methan, isomere Xylilendiamine, Äthylendiamin, Hexamethylendiamin sowie 1,12-Diaminododecan.

Besonders geeignet sind das 4,4-Diaminodiphenylmethan, das 3,3-Dimethyl-4,4-diaminodiphenylmethan und Xylilendiamin.

Für die Herstellung von Harnstoffurethanen in Lösung sind polare aprotische Lösungsmittel notwendig, als solche kommen in Frage :

Dimethylsulfoxid, Hexamethylphosphorsäuretriamid,
NN-Dimethylformamid, N,N,N',N'-Tetramethylharnstoff
NN-Dimethylacetamid,
N-Methylpyrrolidon,
N-Butylpyrrolidon.

Durch den Einsatz von völlig ausreagierten Harnstoffurethanen in dem erfindungsgemässen Thixotropiemittel entfällt der in der vorerwähnten DE-PS 1 805 693 angegebene Überschuß an Amin. Dies ist als Vorteil zu betrachten, da der Überschuß von Aminen zu negativen Erscheinungen im Überzugsmittel führen kann, wie Gelierung von carboxylgruppenhaltigen Bindemitteln, Trocknungsverzögerung von oxidativ trocknenden Bindemitteln, Verfärbung des Anstrichfilms, sowie durch Katalyse Überkondensation bei Einbrennsystemen.

Die in den DE-AS 2 359 923 und DE-AS 2 360 019 beschriebenen Verfahren zur Thixotropierung von Anstrichstoffen haben diesen o. a. Nachteil erkannt und gehen je nach verwendetem Bindemitteltyp von einem Überschuß an Amin oder aber Isocyanat aus, dieser Überschuß im Bindemittel wird dann jeweils mit entsprechenden Reaktionspartnern wie Epoxiden, monofunktionellen Alkoholen oder Oximen wieder abgefangen.

Durch die erfindungsgemäße Harnstofflösung, die weder freie Isocyanatgruppen noch freie Aminogruppen aufweist, wird dem Anwender ein gewerbehygienisch einwandfreies Produkt in die Hand gegeben, das außerdem keine Nebenreaktionen mit Bindemitteln oder Pigmenten eingehen kann und sowohl während der Herstellung eines Überzugsmittels als auch nachträglich dem fertigen Überzugsmittel zugesetzt werden kann.

Die nachfolgenden Beispiele sollen die Herstellung der erfindungsgemäß verarbeiteten Harnstoffurethane erläutern :

Die Herstellung erfolgt in 2 Schritten, wobei der erste Schritt die Herstellung des Akohol-Diisocyanat-Adduktes beinhaltet, der 2. Schritt die Herstellung der Harnstoffurethane.

Allgemeine Herstellung aller in den Tabellen 1a und 1b aufgeführten 12 Harnstoffurethane

1 Mol Diisocyanat wird im Reaktionsgefäß vorgelegt, 1 Mol der entsprechenden OH-funktionellen Verbindung wird langsam zugetropft und die Reaktion je nach verwendetem Diisocyanat nach bekannten Verfahren zu Ende geführt.

Bei aromatischen Diisocyanaten sollte zweckmäßiger Weise die Temperatur nicht über 40 °C steigen, während bei aliphatischen Diisocyanaten Temperaturen von ca. 100 °C zur Bildung der Monoaddukte

angezeigt sind. In einem zweiten Reaktiongefäß werden 1/2 Mol des entsprechenden Polyamins, und 0,1-2 Mol, vorzugsweise 0,5 Mol, Lithiumchlorid in der entsprechenden Menge des aprotischen Lösungsmittels vorgelöst und dann 1 Mol des im 1. Reaktionsschritt gebildeten Isocyanat-Adduktes zugegeben. Die Reaktion verläuft exotherm.

Es resultieren viskose, klare, gelb-braun gefärbte Hernstoffurethanlösungen mit Feststoffgehalten zwischen 10 und 75 %, vorzugsweise zwischen 40 und 50 %.

Tabelle 1a

| | Isocyanat-Monoaddukte | | | |
|---|---|---|---|---|
| Bei-spiel Nr. | Diisocyanate | Hydroxy-funktionelle Verbindungen | NCO-Geh. % theor. | gef. |
| 1 | Toluylendiisocyanat 65 % 2,4-Isomere | Isotridecanol | 11,3 | 11,1 |
| 2 | Toluylendiisocyanat 65 % 2,4-Isomere | 2-Octyldodecanol | 8,6 | 8,4 |
| 3 | Toluylendiisocyanat 65 % 2,4-Isomere | Butyltetraglykol | 9,9 | 9,8 |
| 4 | Toluylendiisocyanat 65 % 2,4-Isomere | Polyoxyäthylenmonomethyläther MG = 350 | 9,6 | 9,5 |
| 5 | Toluylendiisocyanat 65 % 2,4-Isomere | 2-Hexyldecanol | 9,6 | 9,5 |
| 6 | Toluylendiisocyanat 65 % 2,4-Isomere | 2-Hexyldecanol | 9,6 | 9,5 |
| 7 | Toluylendiisocyanat 65 % 2,4-Isomere | 2-Äthylhexanol | 13,8 | 13,6 |
| 8 | Toluylendiisocyanat 80 % 2,4-Isomere | 2-Hexyldecanol | 9,6 | 9,5 |
| 9 | Toluylendiisocyanat 65 % 2,4-Isomere | Isotridecanol | 11,3 | 11,1 |
| 10 | Toluylendiisocyanat 65 % 2,4-Isomere | Isotridecanol | 11,3 | 11,1 |
| 11 | Hexamethylendiisocyanat | Isotridecanol | 12,4 | 12,0 |
| 12 | Toluylendiisocyanat 65 % 2,4-Isomere | 2-Hexyldecanol | 9,6 | 9,5 |

(Siehe Tabelle 1b Seite 5 f.)

Tabelle 1b

| Isocyanat Monoadd. aus Beisp. Nr. | Harnstoffurethane | | | |
|---|---|---|---|---|
| | Polyamine | LiCl Mol/Mol NHCO-NH | Aprotische Lösungsmittel | % Festkörper |
| 1 | 4,4-Diaminodiphenylmethan | 0,5 | N-Methyl-2-Pyrrolidon | 50 |
| 2 | 3,3'-Dimethyl-4,4'-Diaminodiphenylmethan | 1,0 | N-Methyl-2-Pyrrolidon | 40 |
| 3 | 4,4'-Diaminodiphenylmethan | 0,5 | N-Methyl-2-Pyrrolidon | 40 |
| 4 | 4,4'-Diaminodiphenylmethan | 0,1 | N-Methyl-2-Pyrrolidon | 40 |
| 5 | Xylilendiamin-1,3 | 0,5 | N-Methyl-2-Pyrrolidon | 40 |
| 6 | Hexamethylendiamin | 0,5 | N-Methyl-2-Pyrrolidon | 30 |
| 7 | 4,4'-Diaminodiphenylmethan | 0,5 | N-Methyl-2-Pyrrolidon | 40 |
| 8 | 4,4'-Diaminodiphenylmethan | 0,5 | N-Methyl-2-Pyrrolidon | 60 |
| 9 | 4,4'-Diaminodiphenylmethan | 2,0 | Dimethylformamid | 50 |
| 10 | 4,4'-Diaminodiphenylmethan | 0,5 | Dimethylsulfoxid | 50 |
| 11 | 3,3'-Dimethyl-4,4'-Diaminodiphenylmethan | 0,5 | N-Methyl-2-Pyrrolidon | 40 |
| 12 | 3,3'Dimethyl-4,4'-Diaminodicyclohexylpropan | 0,5 | N-Methyl-2-Pyrrolidon | 50 |

Die in den Beispielen 1-12 aufgeführten Harnstoffurethanlösungen werden in Tabelle 2 auf ihre Fähigkeit untersucht in Lösungsmitteln Gele zu bilden. Je nach Polarität der hergestellten Produkte sind diese fähig in unterschiedlich polaren Lösemitteln Gele aufzubauen.

Dies wird beim Einsatz dieser Produkte ausgenützt, und die Polarität der Produkte auf die Polarität der Bindemittel und der Lösungsmittel abgestimmt.

Erläuterung zu Tabelle 2

Bedeutung der Zeichen :

— ≙ kein Gel
tr. Gel ≙ trübes Gel
kl. Gel ≙ klares Gel

(Siehe Tabelle 2 Seite 6 f.)

Tabelle 2

| | Äthylglykol | Butylglykol | Äthylamyl-keton | Wasser | Xylol | Xylol/Isobutanol 1:9 | 50 % Xylol<br>20 % n-Butanol<br>20 % Methylglykol<br>10 % Cyclohexanon |
|---|---|---|---|---|---|---|---|
| 1 | tr. Gel | tr. Gel | tr. Gel | - | tr. Gel | kl. Gel | tr. Gel |
| 2 | - | - | tr. Gel | - | kl. Gel | kl. Gel | - |
| 3 | kl. Gel | tr. Gel | - | - | - | tr. Gel | kl. Gel |
| 4 | tr. Gel | -- | - | tr. Gel | - | - | - |
| 5 | - | - | kl. Gel | - | kl. Gel | kl. Gel | - |
| 6 | - | - | kl. Gel | - | kl. Gel | kl. Gel | - |
| 7 | tr. Gel | tr. Gel | - | - | - | tr. Gel | tr. Gel |
| 8 | tr. Gel | tr. Gel | tr. Gel | - | kl. Gel | kl. Gel | tr. Gel |
| 9 | tr. Gel | tr. Gel | tr. Gel | - | tr. Gel | tr. Gel | tr. Gel |
| 10 | tr. Gel | tr. Gel | tr. Gel | - | tr. Gel | tr. Gel | tr. Gel |
| 11 | - | tr. Gel | - | - | tr. Gel | tr. Gel | tr. Gel |
| 12 | - | - | - | - | tr. Gel | - | - |

In 2 praxisgerechten Lackrezepturen, bei denen es speziell auf dickschichtigen Auftrag zwecks Korrosionsschutz ankommt, wurden 2 der erfindungsgemäßen Produkte (Beispiel 1 + 5 aus der Tabelle 1) geprüft.

Bei den Lacken handelt es sich einmal um einen Chlorkautschuklack (zu Beispiel 1) und einen Vinylharzlack (zu Beispiel 5).

Die Lacke sind wie folgt aufgebaut :

I. Chlorkautschuk Decklack

Standardprobe mit handelsüblichem Thixotropiemittel

a. Mahlansatz :

| | |
|---|---|
| Pergut S 10/Xylol Lösung (21 %ig) (Bayer) | 20,00 g |
| Weichmacher VP-Kl-2351 (Bayer) | 8,44 g |
| Weichmacher VP-Kl-2357 (Bayer) | 8,44 g |
| Thixatrol ST (NL-Industries) | 1,68 g |
| Eisenoxidgelb 415 | 1,12 g |
| Titandioxid R-KB-2 | 16,84 g |
| Chromoxidgrün GN | 5,98 g |
| EWO 473 ($BaSO_4$) | 22,50 g |
| | 85,00 g |

über die Permühle abgerieben

b. Auflackgut :

| | |
|---|---|
| Pergut S 10/Xylol Lösung (21 %ig) | 115,00 g |
| | 200,00 g |

Die Einarbeitung des hydrierten Rizinusöls (Handelsbezeichnung : Thixatrol ST) ist nicht unproblematisch und mit einem beträchtlichen Arbeitsaufwand verbunden. Das pulverförmige Produkt muß zunächst in der Pergut/Xylol-Lösung auf 40-50 °C erwärmt werden, um eine gute Verteilung des Produktes zu erreichen. Das erfindungsgemäße Produkt nach Beispiel 1 wird entweder dem Mahlansatz mit einer Dosierung von 0,5 und 1 % des 50 %igen Produktes, oder wenn die Möglichkeit besteht nachträglich hohe Scherkräft anzuwenden, dem Lackansatz unter starkem Rühren mit dem Dissolver zugegeben.

II. Vinylharz-Decklack

Standardprobe mit handelsüblichem Thixotropiesystem.
(Hydriertes Rizinusöl/Bentonite)

| | |
|---|---|
| Solvesso 100 (Esso) | 16,46 g |
| Xylol | 41,14 g |
| Kristallöl 21 (Shell) | 24,68 g |
| Laroflex MP 35 (BASF) | 36,00 g |
| Chlorparaffin 50 | 5,40 g |
| Bentone 38/ATU-Paste (NL-Industries/Byk-Mallinckrodt) | 14,40 g |
| Thixatrol ST (NL-Industries) | 0,72 g |
| $TiO_2$R-KB-2 | 25,20 g |
| EWO 473 ($BaSO_4$) | 10,80 g |
| Zinkweiß RS | 3,60 g |
| Talkum AT 1 | 21,60 g |
| | 200,00 g |

Das hydrierte Rizinusöl muß auch hier in Xylol vordispergiert werden unter Einhaltung von Temperaturen zwischen 40-55 °C, erst dann können die anderen Bestandteile der Rezeptur eingearbeitet werden. Das erfindungsgemäße Produkt nach Beispiel 5 wird dem Mahlansatz mit einer Dosierung von 0,5 % und 1,0 % des 40 %igen Produktes zugegeben und der Lack normal weiterverarbeitet.

Die resultierenden Lacke werden dann mit dem Stufenspaltrakel (Norm : ASTM D 823) auf Prüfkarten aufgezogen und senkrecht stehend der Ablauf beobachtet. Es wird die Schichtdicke angegeben bei der noch kein Ablaufen oder Absacken der Schicht bemerkt werden kann.

Der resultierende Film wird visuell auf Homogenität bzw. Stippenbildung durch Seeding beobachtet. Die Prüfung der Haftung wird durch Gitterschnitt-Prüfung mit Tesaabriß (ECCA-Methode Nr. 6) auf Glasplatten durchgeführt.

GT 0 ≙ sehr gute Haftung
GT 5 ≙ sehr schlechte Haftung

Testergebnisse Tab. 3.

Tabelle 3

| | Stufenrakel, Ablauf bei | Gitterschnitt | Visuelle Beurteilung der Oberfläche |
|---|---|---|---|
| <u>Chlorkautschuk I</u> | | | |
| Standard Rezeptur | 90 µm | GT 3 | Stippen - Seeding |
| 0,5 % Beispiel 1 | 200 µm | GT 1 | homogen |
| 1,0 % Beispiel 1 | 350 µm | GT 2 | homogen |
| <u>Vinylharz II</u> | | | |
| Standard Rezeptur | 240 µm | GT 5 | homogen |
| 0,5 % Beispiel 5 | 500 µm | GT 1 | homogen |
| 1,0 % Beispiel 5 | 500 µm | GT 1 | homogen |

Nach Auswertung der Ergebnisse aus Tab. 3 kann erkannt werden, daß die erfindungsgemäßen Produkte wesentliche Vorteile für den Anwender bringen.

## Ansprüche

1. Verfahren zur Herstellung einer als Thixotropie-Mittel wirksamen Harnstoffurethane enthaltenden Lösung, dadurch gekennzeichnet, dass vorzugsweise verzweigte Monohydroxyverbindungen ROH mit Diisocyanaten OCN—R'—NCO in Abwesenheit eines Bindemittels umgesetzt und die gebildeten Monoisocyanat-Addukte mit Polyaminen der Formel $H_2N—R''—NH_2$ in aprotischen Lösungsmitteln in Gegenwart von LiCl zu Harnstoffurethanen der Formel :

R—O—CO—NH—R'—NH—CO—NH—R''—NH—CO—NH—R'—NH—CO—OR

R = $C_nH_{2n+1}$- und $C_mH_{2m+1}(C_pH_{2p}O)_r$-
n = 4-22 ; m = 1-18 ; p = 2-4 ; r = 1-10

R' = 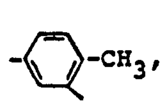, 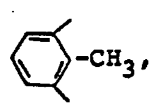, 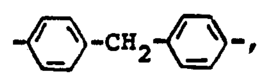, $-(CH_2)_6-$

R'' = 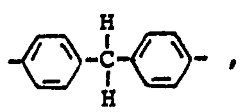, 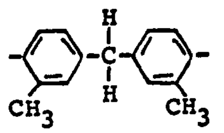,

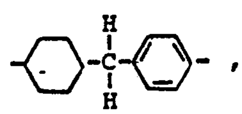, 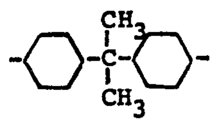

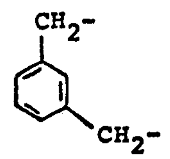

$-CH_2-CH_2-$ , $-(CH_2)_6-$ , $-(CH_2)_{12}-$

umgesetzt werden, wobei der Feststoffgehalt der gebildeten Lösung 10-75 Gew.% beträgt.

2. Verfahren nach Anspruch 1, dadurch gekennzeichnet, dass der Feststoffgehalt der gebildeten Lösung 40-60 Gew.% beträgt.

3. Verfahren nach Anspruch 1-2, dadurch gekennzeichnet, dass als Polyamine primäre und/oder sekundäre aliphatische, cycloaliphatische oder aromatische Amine dienen, die mindestens 2 mit Isocyanaten reagierende Aminogruppen aufweisen.

## Claims

1. Process for preparing a thixotropic agent consisting of a urea urethanes containing solvent comprising the steps of reacting preferably branched chain mono-hydroxy compounds ROH with di-isocyanates OCN—R'—NCO in absence of a bonding agent and converting the formed mono-isocyanate adducts with polyamines of the formula $H_2N—R''—NH_2$ in aprotic solvents in the presence of LiCl into urea urethanes of the following formula :

R—O—CO—NH—R'—NH—CO—NH—R''—NH—CO—NH—R'—NH—CO—OR

R = $C_nH_{2n+1}$- et $C_mH_{2m+1}(C_pH_{2p}O)_r$-
n = 4-22 ; m = 1-18 ; p = 2-4 ; r = 1-10

R' = 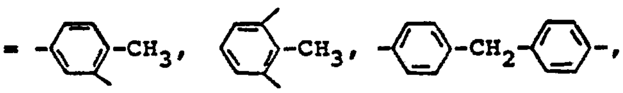 , $-(CH_2)_6-$

R" = 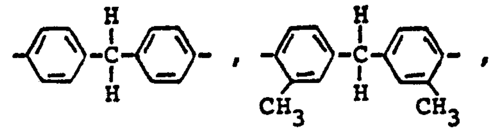

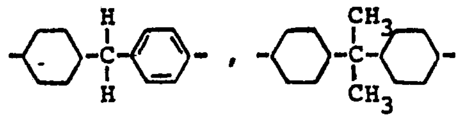

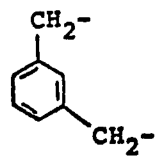

$-CH_2-CH_2-$ , $-(CH_2)_6-$ , $-(CH_2)_{12}-$

said aprotic solvents having a solid content of from 10-75 wt.%.

2. Process according to claim 1, wherein the solids content of the formed solvent is 40-60 wt.%.

3. Process according to claims 1-2, wherein the polyamines are primary and/or secondary aliphatic, cycloaliphatic or aromatic amines which have at least two amine groups for reacting with the isocyanates.

**Revendications**

1. Procédé pour la préparation d'une solution contenant des urée-uréthannes actifs comme agents thixotropants, caractérisé en ce qu'on fait réagir de préférence des composés ROH monohydroxylés ramifiés avec des di-isocyanates OCN—R'—NCO en l'absence d'un liant et on fait réagir les produits d'addition de monoisocyanate formés avec des polyamines de formule $H_2N—R''—NH_2$ dans un solvant aprotique en présence de LiCl pour obtenir des urée-uréthannes de formule :

R—O—CO—NH—R'—NH—CO—NH—R"—NH—CO—NH—R'—NH—CO—OR

R = $C_nH_{2n+1}$- et $C_mH_{2m+1}(C_pH_{2p}O)_r$-
n = 4-22 ; m = 1-18 ; p = 2-4 ; r = 1-10

R' = 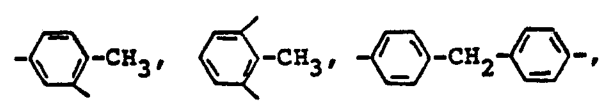 , $-(CH_2)_6-$

R" = 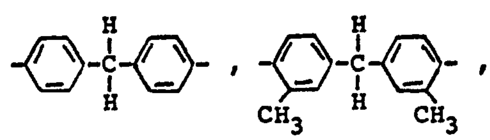

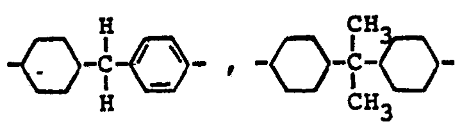

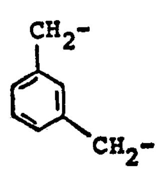

$-CH_2-CH_2-$ , $-(CH_2)_6-$ , $-(CH_2)_{12}-$

auquel cas la teneur en solide de la solution formée est comprise entre 10 et 75 % en poids.

2. Procédé selon la revendication 1, caractérisé en ce que la teneur en solide de la solution formée est comprise entre 40 et 60 % en poids.

3. Procédé selon l'une des revendications 1 ou 2, caractérisé en ce que, comme polyamines, on utilise des amines primaires et/ou secondaires aliphatiques, cycloaliphatiques ou aromatiques, qui possèdent au moins 2 groupes amino réagissant avec des isocyanates.